Europäisches Patentamt

European Patent Office

Office européen des brevets

(19)

(11) Veröffentlichungsnummer: **0 292 472 B1**

# EUROPÄISCHE PATENTSCHRIFT

(12)

(45) Veröffentlichungstag der Patentschrift:
30.10.91 Patentblatt 91/44

(51) Int. Cl.⁵: **A61J 1/00**

(21) Anmeldenummer: 88890127.9

(22) Anmeldetag: 19.05.88

(54) Set zur Bereitstellung und Applikation eines Gewebeklebstoffes.

(30) Priorität: 21.05.87 AT 1304/87

(43) Veröffentlichungstag der Anmeldung:
23.11.88 Patentblatt 88/47

(45) Bekanntmachung des Hinweises auf die
Patenterteilung:
30.10.91 Patentblatt 91/44

(84) Benannte Vertragsstaaten:
AT BE CH DE ES FR GB IT LI LU NL SE

(56) Entgegenhaltungen:
EP-A- 0 037 393
EP-A- 0 148 116
EP-A- 0 156 098

(73) Patentinhaber: IMMUNO Aktiengesellschaft
Industriestrasse 67
A-1221 Wien (AT)

(72) Erfinder: Redl, Heinz, Dr.
Windmühlgasse 7
A-1060 Wien (AT)
Erfinder: Habison, Georg, Dr.
Taubstummengasse 15/12
A-1040 Wien (AT)

(74) Vertreter: Wolfram, Gustav, Dipl.-Ing.
Schwindgasse 7 P.O. Box 205
A-1041 Wien (AT)

## Beschreibung

Die Erfindung betrifft ein Set zur Bereitstellung und Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen von Lösungen der Proteine und blutgerinnungsfördernder Gerinnungsfaktoren in situ gebildet wird, mit einer Mehrzahl von in jeweils eine durchgehende Bohrung aufweisenden Konussen endigenden Spritzenkörpern, vorteilhaft genormten Einwegspritzenkörpern aus Kunststoffmaterial, und gegebenenfalls einer Halteeinrichtung für die Spritzenkörper.

Vorrichtungen dieser Art werden in der EP-A-0037393 und EP-A-0156098 beschrieben. Als Komponenten können einerseits eine Faktor XIII und Fibrinogen enthaltende Proteinlösung (Gewebeklebstoff) und andererseits eine Thrombin enthaltende Lösung verwendet werden. Diese Komponenten werden in einem die Konusse der Spritzenkörper verbindenden Sammelkopf mit aufgesetzter Mischkanüle vermischt und auf die zu behandelnde bzw. zu schützende Wundstelle aufgebracht.

Bei Durchführung von Gewebeklebungen bzw. -behandlungen werden üblicherweise die Proteinlösung und die Thrombinlösung durch Auflösen von Lyophilisaten erhalten. Dabei kann sich insbesondere bei einem chirurgischen Notfall die Schwierigkeit ergeben, daß die Bereitung der Proteinlösung infolge der schweren Löslichkeit dieser Komponente eine unerwünscht lange Zeit in Anspruch nimmt. Es ist zwar möglich, die Auflösungsdauer der Proteinkomponente durch Verwendung einer größeren Menge von Lösungsmittel zu verkürzen, jedoch wird bei der Mischung der Proteinlösung mit der Thrombinlösung auch die clottierende Mischung so stark verdünnt, daß eine signifikante Verringerung der Reißfestigkeit der Klebung als Folge auftritt.

Eine weitere Schwierigkeit besteht darin, daß beim Auflösen unter Luftzutritt eine Schaumbildung eintritt, welche die Applikation erheblich verzögert und sogar einen teilweisen Abbau der Proteine zu Folge haben kann.

Die Erfindung bezweckt die Vermeidung dieser Schwierigkeit und stellt sich die Aufgabe, ein Set zur Bereitstellung und Applikation eines Gewebeklebstoffes zur Verfügung zu stellen, bei welchem bei der Bereitung der Gewebeklebstofflösung aus einem Lyophilisat die Auflösungsdauer verkürzt wird, ohne eine Schaumbildung in Kauf nehmen zu müssen ; und welches eine unmittelbare Applikation der Gewebeklebstofflösung möglich macht.

Diese Aufgabe wird erfindungsgemäß durch ein Bereitstellungs- und Applikationsset gelöst, welches dadurch gekennzeichnet ist,

a) daß jeweils vier Spritzenkörper paarweise zu einer Einheit vereinigt sind, wobei in einem Spritzenkörper des ersten Paares ein Lyophilisat des Proteins und in dem anderen Spritzenkörper des ersten Paares die notwendige Flüssigkeit zum Auflösen des Protein-Lyophilisates eingebracht sind ; und in einem Spritzenkörper des zweiten Paares ein Lyophilisat eines proteolytischen Enzyms und in dem anderen Spritzenkörper des zweiten Paares die notwendige Flüssigkeit zum Auflösen des proteolytischen Enzyms eingebracht sind,

b) daß entweder die einen Förderkanal aufweisenden Konusse jedes Paares der Spritzenkörper integral miteinander verbunden sind, wobei in den verbundenen Förderkanälen ein temporärer Verschluß, insbesondere eine Membran, vorgesehen ist oder die einen Förderkanal aufweisenden Konusse jedes Paares durch ein an sich bekanntes Kupplungsstück miteinander verbunden sind, welches Kupplungsstück einen temporären Verschluß aufweist,

c) die Inhalte jedes Paares der Spritzenkörper durch Druckanwendung über die Förderkanäle zwecks Auflösung der Lyophilisate austauschbar, förderbar und mischbar sind und

d) daß die Spritzenkörper jedes Paares von einander bzw. vom Kupplungsstück trennbar sind und

e) auf die Konusse der jeweils eine Lösung enthaltenden Spritzenkörper in bekannter Weise eine Applikationsvorrichtung, insbesondere ein Aufsteckkopf mit einer Mischkanüle, aufsetzbar ist.

Aus der EP-A1 0148116 ist ein rohrähnliches Kupplungsstück bekannt, mit dem zwei Spritzenkörper, von denen einer mit Mikrokapseln und der andere mit einer Lösungsflüssigkeit für diese Mikrokapseln gefüllt ist, miteinander verbindbar sind. Das Kupplungsstück weist keinen temporären Verschluß auf.

Im erfindungsgemäßen Set können die Spritzenkörper jedes Paares einander gegenüberliegend oder nebeneinander angeordnet sein.

Nach einer bevorzugten Ausführungsform sind in den die Lyophilisate enthaltenden Spritzenkörpern Entlüftungsöffnungen vorgesehen.

Bei Verwendung eines Kupplungsstückes weist dieses zweckmäßig eine Entlüftungseinrichtung auf, die vorzugsweise als Dreiweghahn ausgebildet ist.

Vorteilhaft stehen die die Lyophilisate enthaltenden Spritzenkörper unter Ausschluß von Luft, zweckmäßig unter einer Gasatmosphäre, die von der entstehenden Lösung absorbierbar ist, vorzugsweise $CO_2$.

Nach einer anderen Ausführungsform der Erfindung können die Spritzenkörper als aus Kunststoff gefertigte komprimierbare Beutel oder Ampullen (bottle pack) ausgebildet sein.

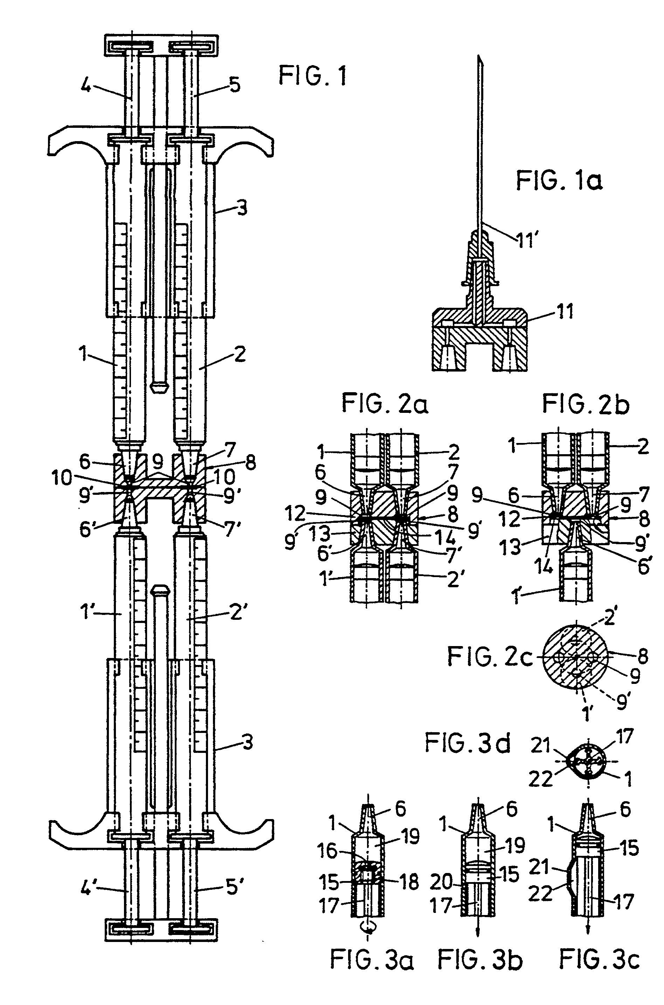

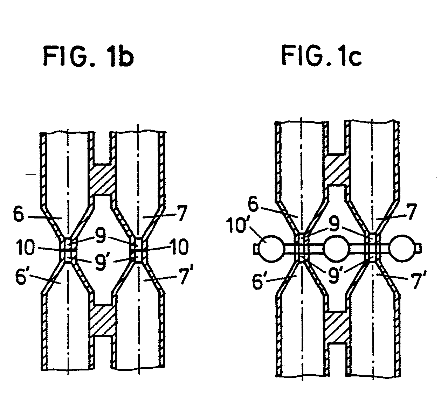

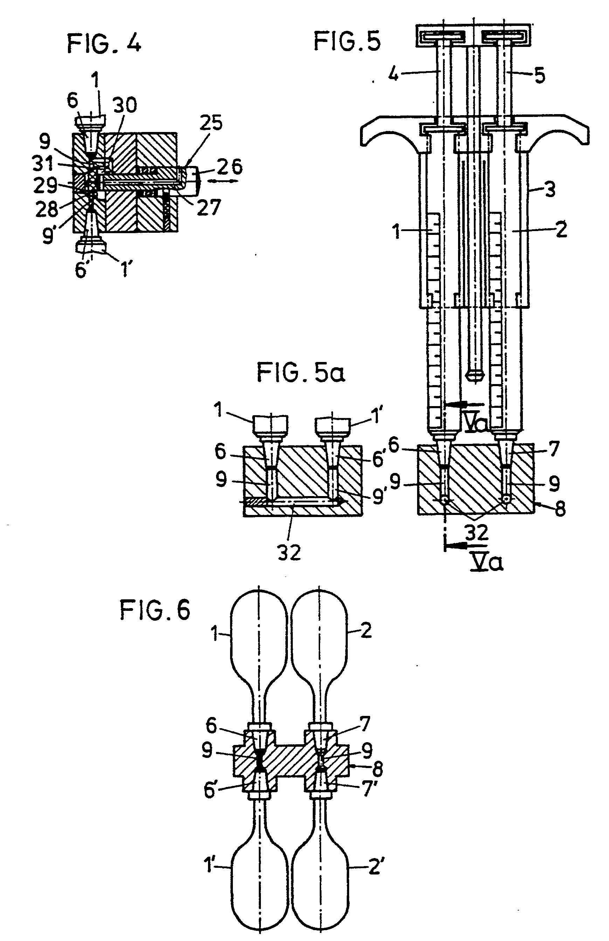

Das Set gemäß der Erfindung ist in der Zeichnung näher erläutert, wobei Fig. 1 eine teilweise geschnittene Gesamtansicht des Sets darstellt. Aus Fig. 1a ist eine Mischkanüle ersichtlich und aus den Fig. 1b und 1c die integral, d.h. fix miteinander verbundenen Konusse der paarweise gegenüberliegenden Spritzenkörper. In den Fig. 2a, 2b und 2c ist ein Kupplungsstück zur Verbindung von paarweise gegenüberliegenden Spritzenkörpern im Axialschnitt (Fig. 2a, 2b) sowie im Schnitt senkrecht hierzu (Fig. 2c) dargestellt und die Fig. 3a, 3b, 3c und 3d erläutern Entlüftungseinrichtungen an Spritzenkörpern, ebenfalls in Schnittdarstellung. Fig. 4 veranschaulicht ein geschnitten dargestelltes Dreiwegventil zur wahlweisen Entlüftung bzw. Vermischung der Inhalte gegenüberliegender Spritzenkörper ; die Fig. 5, 5a zeigen eine Ausführungsform, bei welcher die Spritzenkörper eines Paares nebeneinander angeordnet sind, und Fig. 6 stellt eine abgewandelte Ausführungsform eines Sets gemäß der Erfindung in zu Fig. 1 analoger Darstellung dar, worin die Spritzenkörper als komprimierbare Beutel ausgebildet sind.

In Fig. 1 sind mit 1 und 1' zwei einander gegenüberliegende Spritzenkörper eines ersten Paares bezeichnet, wobei der Spritzenkörper 1 zur Aufnahme des Lyophilisates eines Gewebekleberproteins und der Spritzenkörper 1' zur Aufnahme einer Löseflüssigkeit bestimmt ist. Mit 2 ist ein Spritzenkörper des zweiten Paares bezeichnet, der zur Aufnahme eines Lyophilisates des proteolytischen Enzyms (Thrombin) bestimmt ist, und der gegenüberliegende Spritzenkörper 2' des zweiten Paares dient zur Aufnahme einer Löseflüssigkeit. Die Spritzenkörper 1, 1', 2, 2' sind als genormte Einwegspritzenkörper aus Kunststoff ausgebildet, sie sind gemeinsam in die Halteeinrichtung 3 eingesetzt.

In allen Spritzenkörpern sind mittels Kolbenstangen 4, 4', 5, 5' Kolben geführt. Die Konusse 6, 6' des ersten Paares und 7, 7' des zweiten Paares sind durch ein Kupplungsstück 8 miteinander verbunden, welche Konusse Förderkanäle 9 und 9' aufweisen, durch die die Flüssigkeit aus den Spritzenkörpern 1', 2' in die gegenüberliegenden Spritzenkörper 1, 2 hin- und zurückgefördert werden kann. Die Konusse 6, 6' und 7, 7' der beiden Paare können auch integral über die beiden Förderkanäle 9 und 9' — wie in den Fig. 1b und 1c dargestellt — miteinander verbunden sein. In den Kanälen 9, 9' ist ein temporärer Verschluß vorgesehen, der vorteilhaft als eine unter dem Flüssigkeitsdruck zerreißbare Membran 10 ausgebildet ist. Im Falle einer integralen Verbindung der Konusse 6, 6' und 7, 7' kann der temporäre Verschluß auch durch Quetschung der Förderkanäle mittels der Quetschklemme 10' oder durch Abwinkeln gewährleistet werden.

Durch abwechselnde Betätigung der Kolbenstangen 4, 4' und 5, 5' wird die Membran 10 zerstört und die Löseflüssigkeit über die Förderkanäle 9, 9' hin- und hergefördert, wobei der Inhalt der gegenüberliegenden Spritzenkörper ausgetauscht und in kurzer Zeit eine klare Lösung der Lyophilisate erhalten wird. Nach erfolgter Mischung werden die die fertigen Lösungen enthaltenden Spritzenkörper 1', 2' aus dem Kupplungsstück 8 herausgezogen bzw. bei der integralen Verbindung der Konusse 6, 6' und 7, 7' entweder durch Abbrechen an einer mittigen Sollbruchstelle an den Förderkanälen 9, 9' oder — im Falle eines Quetschverschlusses — durch Abschneiden an einer mittigen Stelle der Förderkanäle 9, 9' von den Spritzenkörpern 1 und 2 getrennt und ein Aufsteckkopf 11 mit einer Kanüle 11' — wie in Fig. 1a dargestellt — auf die Konusse 6', 7' aufgesetzt, worauf durch gleichzeitige Betätigung der Kolbenstangen 4', 5' die Applikation des Gewebeklebstoffes erfolgen kann.

In den Fig. 2a bis 2c ist eine abgeänderte Ausführungsform eines Kupplungsstückes 8 dargestellt, wobei dieses Kupplungsstück als Drehschieber ausgebildet ist. Durch Verdrehen des Teiles 12 gegenüber dem Teil 13 wird, wie in Fig. 2a dargestellt, der Durchfluß durch die Kanäle 9, 9' freigegeben, während bei einer Drehung um 90° der Durchfluß, wie in Fig. 2b dargestellt, gesperrt ist. Mit 14 ist eine Dichtung zwischen den Teilen 12 und 13 bezeichnet.

In den Fig. 3a bis 3d sind Varianten von Entlüftungseinrichtungen dargestellt. Der Kolben 15 in Fig. 3a enthält eine Bohrung 16. Diese Bohrung 16 kann durch Verdrehen der Kolbenstange 17 innerhalb des Gewindes 18 in Dichtstellung gebracht werden, wie in Fig. 3a dargestellt. Bei Zurückdrehen des Kolbens in Gegenrichtung wird die Bohrung freigegeben und die Luft kann aus dem Raum 19 oberhalb des Kolbens nach unten entweichen. Dies geschieht dann, wenn die Löseflüssigkeit aus dem (nicht dargestellten) Spritzenkörper 1' durch den Förderkanal 9 eingedrückt wird.

Bei der Ausführungsform nach Fig. 3b ist in der Wand des Spritzenkörpers 1 eine Entlüftungsbohrung 20 vorgesehen, die, wenn der Kolben in Pfeilrichtung zurückgezogen wird, freigegeben wird.

In den Fig. 3c und 3d ist in der Wand des Spritzenkörpers 1 eine Erweiterung 21 vorgesehen, so daß ein Ringraum 22 entsteht, über den beim Zurückziehen des Kolbens 15 ein Bypass für die ausgedrückte Luft freigegeben wird.

In Fig. 4 ist eine Vorrichtung zur wahlweisen Entlüftung bzw. Vermischung der Inhalte von Spritzenkörpern 1, 1' gezeigt, wobei ein Dreiwegventil 25 in die Förderkanäle 9, 9' eingeschaltet ist. Das Dreiwegventil 25 besteht aus dem in Pfeilrichtung hin- und herbeweglichen Kolben 26, der eine nach außen führende Bohrung 27 aufweist. Am vorderen Ende des Kolbens 26 ist eine konusförmige Gummidichtung 28 vorgesehen, die in den in gleicher Weise konisch ausgebildeten Raum 29 zwischen den Förderkanälen 9, 9' paßt. Vom Förderkanal 9 zweigt eine Bohrung 30 ab, die in die Bohrung 27 mündet.

Wie aus der Zeichnung ersichtlich, kann durch Bewegen des Kolbens 26 in der Doppelpfeilrichtung wahlweise die Verbindung zwischen den Förderkanälen 9, 9' gesperrt werden, in welchem Fall die Leitung 30 freigegeben wird, durch die Luft aus dem Spritzenkörper 1 in die Bohrung 27 und von dort nach außen gedrückt wird, oder es kann durch Zurückziehen des Kolbens und des Dichtungskonus 28 der Durchgang zwischen den Förderkanälen 9 und 9' freigegeben werden, wobei der Dichtungskonus gegen den Anschlag 31 zur Anlage kommt und den Kanal 30 sperrt.

Die Funktion der Vorrichtung ist die, daß nach Fluten des das Lyophilisat enthaltenden Spritzenkörpers 1 mit dem Lösungsmittel vor dem eigentlichen Lösungsvorgang die im Spritzenkörper 1 enthaltene Luft durch die Kanäle 30 und 26 entfernt wird. Bei dem darauffolgenden eigentlichen Durchmischungs- und Lösungsvorgang wird jede Schaumbildung vermieden.

In den Fig. 5 und 5a ist eine Ausführungsform gezeigt, bei der die Spritzenkörper 1 und 1' eines Paares nicht gegenüber-, sondern nebeneinanderliegen. Die Förderkanäle 9, 9' sind durch eine Verbindungsbohrung 32 verbunden, wobei, wie in Fig. 1 dargestellt, eine zerreißbare Membran vorgesehen sein kann.

Eine abgeänderte Ausführungsform des erfindungsgemäßen Sets ist in Fig. 6 dargestellt, wobei die Spritzenkörper 1, 1', 2, 2' als komprimierbare Beutel bzw. Ampullen aus Kunststoffmaterial ausgebildet sind. Diese Ampullen endigen in gleicher Weise, wie in Verbindung mit Fig. 1 beschrieben, in Konussen 6, 6' bzw. 7, 7' und sind durch ein Kupplungsstück 8, welches Förderkanäle 9 aufweist, miteinander zu einer Einheit verbunden. Auch hier kann in den Förderkanälen eine unter Flüssigkeitsdruck zerreißbare Membran oder ein sonstiger temporärer Verschluß vorgesehen sein, wie in Verbindung mit Fig. 2 beschrieben. Im übrigen ist die Funktion die gleiche wie früher beschrieben.

Das erfindungsgemäße Set wird beispielsweise in folgender Weise benutzt :

In Spritzenkörpern gemäß Fig. 1, mit einem Inhalt von 2 ml, werden in einen Spritzenkörper 1125 bis 200 mg lyophilisiertes Kleberproteinkonzentrat eingebracht, der Spritzenkörper unter $CO_2$-Gas gesetzt und in das Kupplungsstück 8 eingesetzt. In den Spritzenkörper 2 werden in gleicher Weise 15 bis 30 mg einer lyophilisierten Thrombin-Präparation, ebenfalls unter $CO_2$-Atmosphäre, eingebracht und dieser wird ebenfalls in das Kupplungsstück gesetzt. In den Spritzenkörper 1' wird 1 ml Aprotininlösung (3000 KIE) und in den Spritzenkörper 2' 1 ml einer Calciumchloridlösung (40 mMol/l) gefüllt ; dann werden auch diese Spritzenkörper in das Kupplungsstück gesetzt. Nun werden durch Drücken der Kolben der beiden mit Lösung gefüllten Spritzenkörper 1', 2' die Flüssigkeiten in die mit Lyophilisat gefüllten Spritzenkörper 1, 2 übergeführt ; durch mehrmalige Wiederholung dieses Vorganges werden die Inhalte der gegenüberliegenden Spritzenkörper 1, 2 bzw. 1', 2' ausgetauscht und vermischt. Nach etwa 120 sec dieses Förder- und Mischvorganges ist das hochkonzentrierte Kleberprotein unter Vermeidung von Schaumbildung zu einer klaren Lösung gelöst. Das $CO_2$-Gas wurde von der Proteinlösung bzw. der Thrombinlösung absorbiert.

Wenn ohne $CO_2$-Atmosphäre in den Spritzenkörpern 1 und 2 gearbeitet wird, sind Entlüftungseinrichtungen in den Spritzenkörpern 1 vorzusehen, wie in Fig. 3 dargestellt, damit die ursprünglich über den Lyophilisaten in den Spritzenkörpern 1 und 2 vorhandene Luft entfernt wird. Im übrigen ist die Funktionsweise die gleiche.

## Patentansprüche

1. Set zur Bereitstellung und Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen von Lösungen der Proteine und blutgerinnungsfördernder Gerinnungsfaktoren gebildet wird, mit einer Mehrzahl von in jeweils eine durchgehende Bohrung aufweisenden Konussen (6, 6' ; 7, 7') endigenden Spritzenkörpern (1, 1', 2, 2'), vorteilhaft genormten Einwegspritzenkörpern aus Kunststoffmaterial, und gegebenenfalls einer Halteeinrichtung (3) für die Spritzenkörper (1, 1', 2, 2'), dadurch gekennzeichnet,

a) daß jeweils vier Spritzenkörper (1, 1', 2, 2') paarweise zu einer Einheit vereinigt sind, wobei in einem Spritzenkörper (1) des ersten Paares ein Lyophilisat des Proteins und in dem anderen Spritzenkörper (1') des ersten Paares die notwendige Flüssigkeit zum Auflösen des Protein-Lyophilisates eingebracht sind ; und in einem Spritzenkörper (2) des zweiten Paares ein Lyophilisat eines proteolytischen Enzyms und in dem anderen Spritzenkörper (2') des zweiten Paares die notwendige Flüssigkeit zum Auflösen des proteolytischen Enzyms eingebracht sind,

b) daß entweder die einen Förderkanal (9, 9') aufweisenden Konusse (6, 6' bzw. 7, 7') jedes Paares (1, 1' bzw. 2, 2') der Spritzenkörper integral miteinander verbunden sind, wobei in den verbundenen Förderkanälen (9, 9') ein temporärer Verschluß, insbesondere eine Membran (10), vorgesehen ist, oder die einen Förderkanal (9, 9') aufweisenden Konusse (6, 6' bzw. 7, 7') jedes Paares durch ein an sich Bekanntes Kupplungsstück (8) miteinander verbunden sind, welches Kupplungsstück einen temporäten Verschluß (10) aufweist,

c) die Inhalte jedes Paares der Spritzenkörper (1, 1′ bzw. 2, 2′) durch Druckanwendung über die Förderkanäle (9, 9′) zwecks Auflösung der Lyophilisate austauschbar, förderbar und mischbar sind und

d) daß die Spritzenkörper (1, 1′ bzw. 2, 2′) jedes Paares von einander bzw. vom Kupplungsstück (8) trennbar sind und

e) auf die Konusse (6′, 7′) der jeweils eine Lösung enthaltenden Spritzenkörper (1′, 2′) in bekannter Weise eine Applikationsvorrichtung, insbesondere ein Aufsteckkopf (11) mit einer Mischkanüle (11′), aufsetzbar ist.

2. Set nach Anspruch 1, dadurch gekennzeichnet, daß im Set die Spritzenkörper (1, 1′ bzw. 2, 2′) jedes Paares einander gegenüberliegend oder nebeneinander angeordnet sind.

3. Set nach Anspruch 1, dadurch gekennzeichnet, daß in den die Lyophilisate enthaltenden Spritzenkörpern (1, 2) Entlüftungsöffnungen (16 ; 20 ; 22) vorgesehen sind.

4. Set nach Anspruch 1 oder 2, dadurch gekennzeichnet, daß das Kupplungsstück (8) eine Entlüftungseinrichtung, die vorzugsweise als Dreiwegventil (25) ausgebildet ist, aufweist.

5. Set nach einem der Ansprüche 1 bis 4, dadurch gekennzeichnet, daß die die Lyophilisate enthaltenden Spritzenkörper (1, 2) unter Ausschluß von Luft, zweckmäßig unter einer Gasatmosphäre, die von der entstehenden Lösung absorbierbar ist, vorzugsweise $CO_2$ stehen.

6. Set nach Anspruch 1, dadurch gekennzeichnet, daß die Spritzenkörper (1, 2, 1′, 2′) als aus Kunststoff gefertigte komprimierbare Beutel oder Ampullen ausgebildet sind.

## Claims

1. A set for providing and applying a tissue adhesive based on human or animal proteins for seamlessly or seamsupportingly connecting human or animal tissue or organ parts, for sealing wounds, stopping bleedings and the like, which tissue adhesive is formed by uniting solutions of the proteins and of blood-clot promoting coagulation factors, comprising a plurality of syringe bodies (1, 1′, 2, 2′) ending in coni (6, 6′ ; 7, 7′) each including a through bore, advantageously standardized disposable syringe bodies of synthetic material, and, if desired, a holding means (3) for the syringe bodies (1, 1′, 2, 2′), characterized in that

a) four syringe bodies (1, 1′, 2, 2′) are each pairwisely combined to a unit, a lyophilisate of the protein being contained in a syringe body (1) of the first pair and the liquid necessary to dissolve said protein lyophilisate being contained in the other syringe body (1′) of the first pair ; and a lyophilisate of a proteolytic enzyme being contained in a syringe body (2) of the second pair and the liquid necessary to dissolve said proteolytic enzyme being contained in the other syringe body (2′) of said second pair,

b) either the coni (6, 6′ ; 7, 7′) of each pair (1, 1′ ; 2, 2′) of the syringe bodies, including conveying channels (9, 9′), respectively are integrally connected with each other, a temporary closure means, in particular a membrane (10), being provided in the connected conveying channels (9, 9′), or the coni (6, 6′ ; 7, 7′) of each pair, including conveying channels (9, 9′), respectively are connected with each other by a coupling piece (8) known per se, which coupling piece comprises a temporary closure means (10),

c) the contents of each pair of the syringe bodies (1, 1′ ; 2, 2′), by applying pressure, are exchangeable, conveyable and miscible via the conveying channels (9, 9′) for the purpose of dissolving the lyophilisates,

d) the syringe bodies (1, 1′ ; 2, 2′) of each pair are separable from each other or from the coupling piece (8), respectively, and

e) an application means, in particular a slip-on head (11) including a mixing cannula (11′), is placeable on the coni (6′, 7′) of the syringe bodies (1′, 2′) each containing a solution, in a manner known per se.

2. A set according to claim 1, characterized in that, within the set, the syringe bodies (1, 1′ ; 2, 2′) of each pair are arranged opposite each other or adjacent each other.

3. A set according to claim 1, characterized in that vents (16 ; 20 ; 22) are provided in the syringe bodies (1, 2) containing the lyophilisates.

4. A set according to claim 1 or 2, characterized in that the coupling piece (8) comprises a ventilation means, preferably designed as a three-way valve (25).

5. A set according to any one of claims 1 to 4, characterized in that the syringe bodies (1, 2) containing the lyophilisates are under the exclusion of air, suitably under a gas atmosphere to be absorbed by the solution forming, preferably $CO_2$.

6. A set according to claim 1, characterized in that the syringe bodies (1, 2, 1′, 2′) are designed as compressible bags or ampoules made of synthetic material.

## Revendications

1. Equipement pour la préparation et l'application d'une colle tissulaire, à base de protéines humaines ou animales pour réunir en l'absence de suture ou en en renforçant la suture des fragments de tissus ou d'organes humains ou animaux, pour refermer des plaies, pour assurer l'hémostase, etc., la colle tissulaire étant formée par la mise en contact de solutions des protéines et de facteurs de coagulation favorisant la coagulation sanguine, composé d'une pluralité de corps de seringues (1, 1', 2, 2') se terminant par des cônes (6, 6', 7, 7') présentant chacun un trou de passage, avantageusement des corps de seringues à jeter normalisés en matière plastique, et le cas échéant d'un dispositif de fixation (3) des corps de seringues (1, 1', 2, 2'), caractérisé en ce que

a) quatre corps de seringues (1, 1', 2, 2') sont réunis par paires en une unité, un lyophilisat de protéine étant introduit dans un corps de seringue (1) de la première paire et le liquide nécessaire à la dissolution du lyophilisat de protéine étant introduit dans l'autre corps de seringue (1') de la première paire ; et un lyophilisat d'une enzyme protéolytique étant introduit dans un corps de seringue (2) de la seconde paire et le liquide nécessaire à la dissolution de l'enzyme protéolytique étant introduit dans l'autre corps de seringue (2') de la seconde paire,

b) soit les cônes (6, 6' et 7, 7') de chaque paire (1, 1' ou 2, 2') de corps de seringues, présentant un canal de transport (9, 9') sont réunis entre eux en un seul bloc, une obturation temporaire, notamment une membrane (10), étant prévue dans les canaux de transport reliés (9, 9'), soit les cônes (6, 6' ou 7, 7') de chaque paire, présentant un canal de transport (9, 9') sont reliés entre eux par un raccord (8) connu en soi, ce raccord présentant une obturation temporaire (10),

c) les contenus de chaque paire de corps de seringues (1, 1' ou 2, 2') sont interchangeables, transportables et miscibles par application d'une pression, par l'intermédiaire des canaux de transport (9, 9') en vue de la dissolution des lyophilisats et

d) les corps de seringues (1, 1' ou 2, 2') de chaque paire sont séparables les uns des autres ou du raccord (8) et

e) un dispositif d'application, notamment une tête rapportée (11) munie d'une canule de mélange (11') est enfichable de façon connue sur les cônes (6', 7') des corps de seringues (1', 2') contenant chacun une solution.

2. Equipement selon la revendication 1, caractérisé en ce que les corps de seringues (1, 1' ou 2, 2') de chaque paire dans l'équipement sont opposés ou côte à côte.

3. Equipement selon la revendication 1, caractérisé en ce qu'il est prévu des évents (16 ; 20 ; 22) dans les corps de seringues (1, 2) contenant les lyophilisats.

4. Equipement selon la revendication 1 ou 2, caractérisé en ce que le raccord (8) présente un dispositif de désaérage ayant de préférence la forme d'une vanne a trois voies (25).

5. Equipement selon l'une des revendications 1 à 4, caractérisé en ce que les corps de seringues (1, 2) contenant les lyophilisats se trouvent a l'abri de l'air, de façon appropriée sous une atmosphère gazeuse, absorbable par la solution formée, de préférence $CO_2$.

6. Equipement selon la revendication 1, caractérisé en ce que les corps de seringues (1, 2, 1', 2') ont la forme de poches ou d'ampoules comprimables, en matière plastique.

FIG. 1

FIG. 1a

FIG. 2a

FIG. 2b

FIG. 2c

FIG. 3d

FIG. 3a

FIG. 3b

FIG. 3c

## FIG. 1b

## FIG.1c

FIG. 4

FIG. 5

FIG. 5a

FIG. 6